# EUROPEAN PATENT SPECIFICATION

(11) **EP 3 103 879 B1**
(45) Date of publication and mention of the grant of the patent: **21.06.2017**
(21) Application number: 15171167.8
(22) Date of filing: 09.06.2015
(51) Int. Cl.: C12P 19/04, C12P 1/04

(54) **EXTRACELLULAR FRUCTANS PRODUCING ACETIC ACID BACTERIAL STRAIN GLUCONOBACTER NEPHELII P1464**
ESSIGSÄUREBAKTERIENSTAMM GLUCONOBACTER NEPHELII P1464 ZUR ERZEUGUNG EXTRAZELLULÄRER FRUCTANE
FRUCTOSANES EXTRACELLULAIRES PRODUISANT DE L'ACIDE ACÉTIQUE PRÉSENTANT UNE ACTIVITÉ BACTÉRIENNE DE GLUCONOBACTER P1464 NEPHELII

(43) Date of publication of application: 14.12.2016
(73) Proprietor: Latvijas Universitate, 1586 Riga (LV)
(72) Inventor: Semjonovs, Pavels, 1024 Riga (LV); Zikmanis, Peteris, 1012 Riga (LV); Sakirova, M Laisana, 1058 Riga (LV); Treimane, LV Rita, 2015 Jurmala (LV); Patetko, Arturs, 1058 Riga (LV); Auzina, Lilija, 1055 Riga (LV)
(74) Representative: Fortuna, Aleksandra

(56) References cited:
- EP-A1- 2 829 612
- EP-A1- 2 876 169
- HERMANN M ET AL: "Cultivation of selected Gluconobacter-strains on cereal substrates and in-situ production of exopolysaccharides", V SYMPOSIUM ON SOURDOUGH / CEREAL FERMENTATION FOR FUTURE FOODS; 10-12 OCTOBER 2012 ; HELSINKI, FINLAND, , 10 October 2012 (2012-10-10), page 87, XP002720334, Retrieved from the Internet: URL:http://www.vtt.fi/inf/pdf/technology/2 012/T50.pdf [retrieved on 2014-02-13]

## Description

### Technical Field

The invention relates to an isolated and identified strain representing acetic acid bacteria species *Gluconobacter nephelii* and producing extracellular fructan-type polysaccharides. This invention also relates to a method for obtaining fructose polymer using acetic acid bacteria *G. nephelii.*

### Background Art

Acetic acid bacteria (AAB) are Gram-negative, obligatory aerobic bacteria, which membrane-associated dehydrogenase catalyses incomplete oxidation of carbohydrates and alcohols [1; 2; 4]. A number of *Acetobacter* and *Gluconobacter* strains are capable to oxidize ethanol to acetic acid, are resistant to acetic acid and ethanol, which is essential to industrial production of acetic acid. In turn bacteria of genus *Acetobacter* are capable to synthesize exopolysaccharides (EPS), especially cellulose [1; 6; 4]. Some *Gluconobacter xylinus* strains are forming heteropolysaccharide (HePS) acetan and the homopolysaccharide (HoPS) levan [9]. HePS, such as xanthan, comprises of different carbohydrate residues, which are intracellularly polymerised by a number of enzymes. HoPS mainly represent polymers of glucose (glucans) or fructose (fructans), which as EPS are synthesized from sucrose by extracellular glycosyl- or fructosyltransferase [6; 7].

Fructans of bacterial origin are fructose polymers with β-(2.6) bonds in the main chain [5; 7; 14; 17]. Fructans are classified as oligosaccharides (FOS) or polysaccharides according to their degree of polymerization. FOS or low-molecular fructans are containing between 3 to 10 monosaccharide units. In turn high-molecular fructans (levan) are containing more than 11 monosaccharide units [1; 8, 13]. Earlier studies have found that levan can be synthesized by organisms belonging to various genera, including *Zymomonas, Streptococcus, Xanthomonas, Bacillus, Erwinia and Pseudomonas* [7 - 9; 13; 14]. It is known that a number of *Acetobacter* strains can also produce levan [1; 6; 8; 12]. Recently it has been demonstrated that bacteria [1; 12] of the genus *Gluconobacter* possess the ability to form fructans. However, despite attempts to optimize conditions for synthesis of levan in different bacterial cultures, the yield of levan is still rather low [5]. Moreover, it is found that fructans may represent only a part of the total EPS. Thus, only 25% of levan are defined in *G. xylinus* overall EPS [8]. *Gluconobacter nephelii* is the relatively newly isolated and identified AAB species [10]. It is still a scarcely studied organism, and it is also not reported of any kind EPS formation in *G. nephelii* bacteria.

Due to their versatile qualities, fructans of microbial origin, especially bacterial origin, have found practical application in a number of spheres. Fructans are applicable as functional food ingredients [6; 15; 16], because they stimulate the physiological and biochemical processes in the human body, promote growth of favourable microflora, especially bifidobacteria, [8; 12], thereby strengthen the organism's immune system. Such polysaccharides are defined as prebiotics [12; 15]. Levan can be used in medicine as a plasma substitute, source of fructose, anti-inflammatory agent, detoxycator, drug activity promoter and prolongator, the immunomodulator having radioprotecting and anti-cancer properties [3; 16; 17]. Besides levan can be used as a thickener, emulsifier, encapsulating agent, as a carrier of color and flavor in food, in pharmaceutical and cosmetic industry [5; 6; 8; 11; 16-18]. Application of levan also is found in nanotechnology for creating novel nanocomposites [17].

Despite varied and obvious functional and technological advantages of fructans, their industrial use is still insufficient, mainly due to relatively high costs. Therefore optimization of levan production, which includes selection of more efficient, e.g. more productive and technologically more perspective cultures and improvement of desired properties for its application to industrial production is now recognized as a promising task of industrial biotechnology [18; 19].

Fructan production and use thereof is disclosed in [26]. The capability of fructan production is also known from [27-28].

### Disclosure of the Invention

The novel extracellular fructans producing acetic acid bacterial strain *Gluconobacter nephelii* P 1464 is proposed, and the products obtained by its use, which products containing strain P 1464 and extracellular fructan-type polysaccharides produced by it.

The proposed strain *Gluconobacter nephelii* is deposited in the Microbial Strain Collection of Latvia under the number P 1464.

The *Gluconobacter nephelii* P 1464 strain cells are rod-shaped (1.1 µm x 1.5 -2.5 µm), even or odd-shaped, rigid, they do not form spores, dye according Gram - negative, oxidase reaction - negative; catalase reaction - positive.

### Brief Description of Drawings

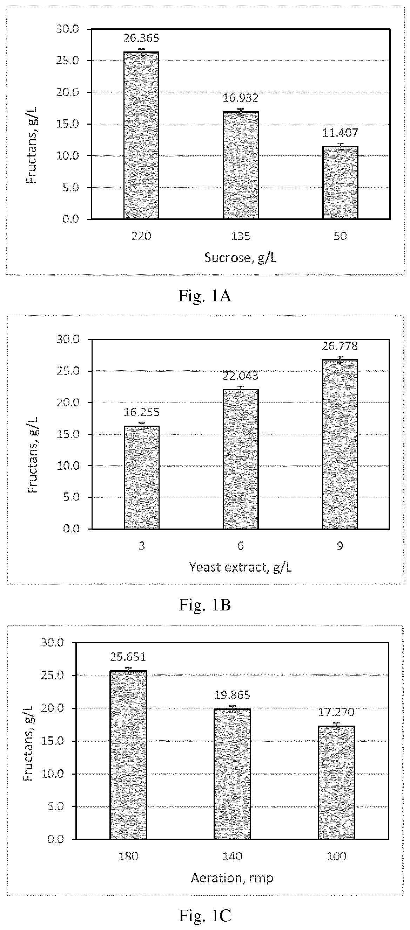

Fig. 1A-1C shows the results of *Gluconobacter nephelii* P 1464 extracellular fructan biosynthesis in different cultivation conditions: 1A - depending on the substrate concentration (concentration of yeast extract 6 g/L, aeration 240 rmp); 1B - depending on yeast extract concentration (concentration of sucrose 135 g/L, aeration 170 rmp); 1C - depending on the aeration intensity (sucrose and yeast extract concentrations, respectively, 150 g/L and 7.5 g/L);

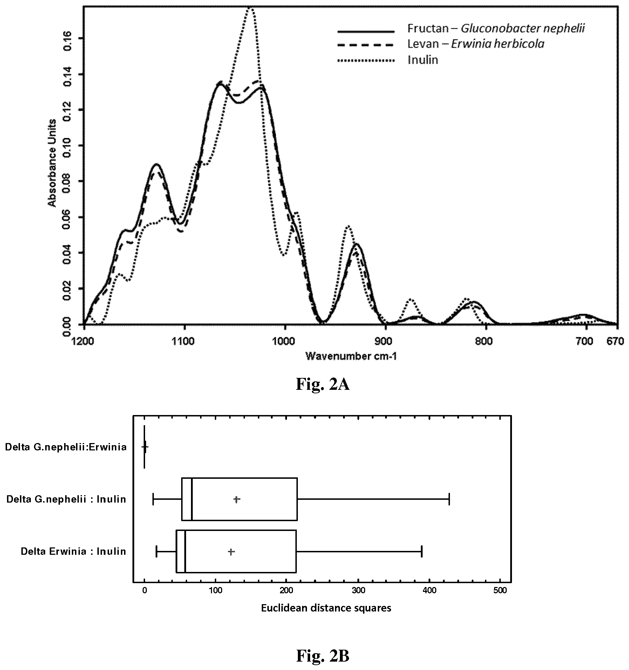

Fig. 2A - fructose polymer infrared (FT-IR) spectra; 2B - statistical evaluation of its "similarity" according to Box-Whisker plot;

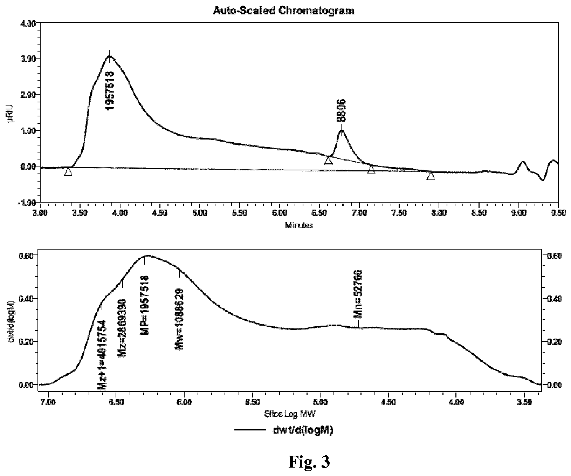

Fig. 3 - the molecular weight distribution of *Gluconobacter nephelii* P 1464 extracellular fructan preparation by using molecular exclusion chromatography (SEC) method; the chromatogram obtained with ACQUITY APC chromatograph (Waters Acquity Advanced Polymer Chromatograph, MA, USA), by using APC AQ-45A+APC AQ125A+APC AQ-450A columns, ACQUITY RI detector and controlling system Empower 3; the mobile phase: Acetonitrile/200 mM NaNO₃ in deionized water at 40°C;

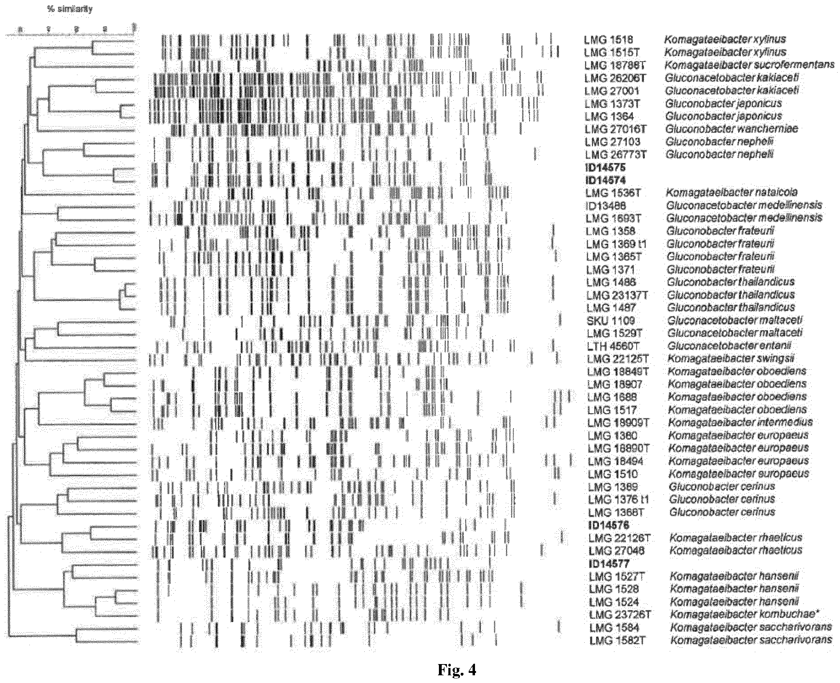

Fig. 4 - the *Gluconobacter nephelii* homology and filogenetic tree, within the proposed P 1464 strain is identified; designated in the figure by ID 14575.

The isolated and identified P 1464 culture is cultivated aerobically at 30°C (not exceeding 32°C) temperature, on the agarized Hestrin - Schramm (HS) medium of following composition (g/L): glucose - 20.0; peptone - 5.0; yeast extract - 5.0; Na₂HPO₄ + 12H₂O - 7.3; citric acid -1.15; MgSO₄ - 0.5; agar - 15.0; pH = 5.6 ± 0.2.

The culture is stored in the HS medium with glycerol additive of 10-15% at -18°C - -20°C temperature. The culture is unfrozen (22°C) and, by using the series dilution method inoculated on agarized HS medium and cultivated aerobically at 30°C (not exceeding 32°C) temperature.

The isolated and identified culture forms extracellular fructans, growing on HS medium, which contain sucrose (50 - 200 g/L) as a sole carbon source at an optimal temperature of 30°C. Amount of fructans, when reaching the peak by setting the stationary phase, depends on concentration of sucrose and yeast extract (3 - 9 g/L) in the medium, as well as of aeration intensity (Fig. 1, A, B and C, respectively). The cultivation medium is centrifuged (10 min at 8000 rpm), in order to separate bacterial cells and fructans, separated from the centrifugate by adding cooled ethanol in the ratio of 1:3 (3 volumes of ethanol and 1 volume of cultivation medium). Fructans are purified by repeatedly precipitation from aqueous solutions with ethanol. Proteins are separated from fructan aqueous solutions by adding 50 mM of CaCl₂ (final concentration 5 mM) by cooling 6 h and centrifugation (at 10000 rpm). The precipitated and purified fructans are dried by lyophilization.

The characteristics of bacterial extracellular fructan formation by its cultivation in bioreactors (Table 1) testifies about the possibility of biotechnological application of the culture.

**Table 1.**

| *G. nephelii* P 1464 biotechnological indexes depending on the substrate and yeast extract (YE) concentration (24 h cultivation in the fermentation facility *Sartorius Q-plus*; at the aeration intensity of 0.25 1/1). | | | | | | |
|---|---|---|---|---|---|---|
| **Variants** | | | | | | |
| **Index** | **120/5 Sucrose/YE** | **170/5** | **220/5** | **120/10** | **170/10** | **220/10** |
| **S₀-S₁ g/L^{a}** | 56,02 | 78.90 | 128.35 | 51.28 | 76.01 | 67.59 |
| **Qp g/(L*h)^{b}** | 2.334 | 3.288 | 5.348 | 2.137 | 3.167 | 2.816 |
| **Yp/s g/g^{c}** | 0.359 | 0.337 | 0.219 | 0.380 | 0.531 | 0.426 |
| **Qx g/(L*h)^{d}** | 0.005304 | 0.005383 | 0.004829 | 0.0057 | 0.005146 | 0.004592 |
| **Yx/s g/g^{e}** | 0.002272 | 0.001637 | 0.000903 | 0.002668 | 0.001625 | 0.00163 |
| **qp g/(g x h)^{f}** | 6.578 | 8.570 | 10.104 | 5.931 | 13.617 | 10.890 |
| **qs g/(g x h)^{g}** | 18.336 | 25.445 | 46.141 | 15.619 | 25.643 | 25.557 |

| | | | | | | |
|---|---|---|---|---|---|---|
| a - overall substrate consumption, So - substrate concentration 0h, S₁ - substrate concentration 24 h b - productivity for fructans c - yield of the product (fructans) from a substrate unit d - productivity by biomass e - yield of biomass from a substrate unit f - the specific rate of the product formation g - the specific rate of the substrate consumption | | | | | | |

*Gluconobacter nephelii* P 1464 is cultivated aerobically, at 30°C, 48h, in the Hestrin - Schramm medium (Schramm M., Hestrin S. Synthesis of cellulose by *Acetobacter xylinum.* 2. Preparation of freeze-dried cells capable of polymerizing glucose to cellulose Biochem J. 1954, 58(2): 345-352), by changing sucrose as the sole carbon source, and yeast extract concentrations in the cultivation medium and aeration intensity.

Fructans quantities are determined gravimetrically [9, 20].
Characteristics of the extracellular fructans:
- *G.nephelii* extracellular polysaccharides, by hydrolysis under acidic conditions (HCl, oxalic acid), the final product is fructose (Table 2);
- spectroscopic data of FT-IS confirm a higher degree of similarity to the bacterial levan (β-2.6 fructan), compared with inulin (β-2.1 fructan) (Fig. 2);
- average molecular weight and rheological indexes of the extracellular fructans correspond to the characteristics of fructans of bacterial origin (levan) (Fig. 3; Table 3).

**Table 2**

| The results of hydrolysis of extracellular fructans synthesized by *Gluconobacter nephelii* P 1464 | | | |
|---|---|---|---|
| Fructan g/L | Monoses ± SD g/L | | Conditions of hydrolysis |
| | Reducing sugars ^{a} | Fructose ^{b} | |
| 18.10 | 18.05 ± 0.07 | 17.50 ± 0.20 | 0.1N HCl (90°C, 3h) |
| 18.20 | 18.15 ± 0.07 | 17.25 ± 0.20 | 0.1N Oxalic acid (100°C, 1h) |
| 18.05 | 18.00 ± 0.07 | 17.40 ± 0.20 | Fructozyme L^{c} (15µl/ml, 60°C, 24h) |

| | | | |
|---|---|---|---|
| ^{a} - DNA method [21]; ^{b} - data obtained by using Megazyme D-Fructose/D-Glucose Assay Kit (*http:*//*secure.megazyme.com*/*D-Fructose-D-Glucose-Assay-Kit*) ^{c} - enzymatic hydrolysis with Fructozyme L [22]. | | | |

**3. Table. Average molecular weight and rheological indexes of Exopolysaccharide (fructan) synthesized by Gluconobacter nephelii P 1464**

| Characteristics | A numeric value +/- SD |
|---|---|
| Number average molecular weight Mn | 53513 +/- 1056 |
| Weight average molecular mass Mw | 1186.28 +/- 138.11 |
| (kDa) | |
| Polydispersity Mw/ Mn | 22.15 +/- 3.03 |
| Intrinsic viscosity η (cL/g) | 0.179 +/- 0.004 |
| Huggins' constant | 0.582 +/- 0.108 |

| | |
|---|---|
| Mw and Mn values are determined by using ACQUITY APC method (Waters Acquity Advanced Polymer Chromatograph, MA, USA), with APC AQ-45A+APC AQ125A+APC AQ-450A columns, ACQUITY RI detector and controlling system Empower 3. The mobile phase: Acetonitrile/200 mM NaNO₃ in deionized water, at 40°C. | |

The system was calibrated with high-molecular dextran standards. For the polysaccharide viscosity measurement a rotational viscometer DV-III Ultra Rheometer was used (Brookfield, MA, USA). The relative viscosity (ηᵣ) was determined for aqueous solutions in the range of exopolysaccharide concentrations 0.5 g/cL - 1.5 g/cL. For the calculation of rheological characteristics Huggins' equation and its graphical form [23] was used.

Infrared spectra of Fructose polymers (Fig. 2A-2B) were compared using the Euclidean distances and its squared values [24] between IR absorption peaks of individual spectrum pairs in areas of "carbohydrates" and the so called "fingerprints" (1200 cm⁻¹ - 600 cm⁻¹) [25]. As fructose polymer standards inulin from dahlia tubers and *Erwinia herbicola* bacterial levan from Sigma- Aldrich (13754 and L8647, respectively) was used. The significance of the difference (B) is confirmed by the criteria of Kruskal-Wallis and Mann-Whitney of nonparametric statistics. FT-IS spectra were taken with a Vertex 70 spectrometer, which was mated with the HTS-XT Microplate Reader (Bruker Optik GmbH, Germany). Samples were dried in 384-hole silicon wafers, at temperature not exceeding 50°C. The average infrared region (4000 - 600 cm⁻¹) was used. Each spectrum was recorded with a resolution of 4 cm⁻¹ as an average of 64 measurements with baseline correction and normalization. For data processing Opus 6.5 software was used.

The offered strain *Gluconobacter nephelii* P 1464 and extracellular fructan-type polysaccharides produced by it can be used for obtaining various products, including food and pharmaceutical products. Thus said fructans producing strain can be used for non-alcoholic beverages, sour milk products and bakery products production, thereby improving the rheological properties of these products, extending the period of validity of bread, giving to all these products functional properties (cholesterol reduction, improvement of digestion) and other.

### Sources of Information

1. Jacob F., Steger S., Vogel R. F. Influence of novel fructans produced by selected acetic acid bacteria on the volume and texture of wear breads. Eur. Food Res. Technol., 2012, 234:493-499.
2. Raspor P. and Goranoviĉ D. Biotechnological applications of acetic acid bacteria. Crit. Rev. Biotechnol., 2008, 28(2):101-24.
3. Biopolymers, Vol. 5. Polysaccharides I. Polysaccharides from Procariotes. Ed. E.J. Vandmme, De Baets and A. Steinbuchel
4. Saichana N., Marsushita K., Adachi O., Frebort I., Frebortova J. Acetic acid bacteria: A group of bacteria with versatile biotechnological applications. Biotech. Adv., 2014, in press
5. Donot F., Fontana A., Baccou J. C., Schorr-Galindo S. Microbial exopolysaccharides: Main examples of synthesis, excretion, genetics and extraction. Carbohydrate Polymers, 2012, 87:951-962.
6. Srikanth R., Siddartha G., Reddy C. H.S.S.S., Harish B.S., Ramaiah M.J., Uppuluri K.B. Antioxidant and anti-inflammatory levan produced from Acetobacter xylinum NCIM2526 and its statistical optimization. Carbohydrate Polymers, 2015, 123:8-16.
7. Han Y. W. and Clarke M. A. Production of fructan (levan) polyfructose polymers using Bacillus polymyxa*.* US 5547863.
8. H. Kornmann, P. Duboc, I. Marison, U. von Stockar. Influence of Nutritional Factors on the Nature, Yield, and Composition of Exopolysaccharides Produced by Gluconacetobacter xylinus I-2281. Appl Environ Microbiol, (2003), Vol. 69, No. 10, p. 6091-6098
9. N. R. Jathore, M. V. Bule, A. V. Tilay, U. S. Annapure. Microbial Levan from Pseudomonas fluorescens: Characterization and Medium Optimization for Enhanced Production. Food Sci. Biotechnol., (2012), 21(4): 1045-1053
10. J. Kommanee, S. Tanasupawat, P. Yukphan, T. Malimas, Y. Muramatsu, Y. Nakagawa, Y. Yamada. Gluconobacter nephelii sp. nov., an acetic acid bacterium in the class Alphaproteobacteria. Int. J. System. Evol. Microbiol., 2011, 61:2117-2122.
11. K. Tajima, N. Uenishi, M. Fujiwara, T. Erata, M. Munekata, M. Takai .The production of a new water-soluble polysaccharide by Acetobacter xylinum NCI 1005 and its structural analysis by NMR spectroscopy. Carbohydrate Research, (1998), 305:117-122.
12. F. Jakob, A. Pfaff, R. Novoa-Carballal, H. Rübsam, T. Becker, R. F. Vogel. Structural analysis of fructans produced by acetic acid bacteria reveals a relation to hydrocolloid function. Carbohydrate Polymers, (2013), 92: 1234-1242.
13. Vincent et. al. Levan-producing *Lactobacillus* strain and method of preparing human or pet food products using the same. US 6932991 B2.
14. T. D. Mays and E. L. Dally. Microbial production of polyfructose. WO 1986004091 A1.
15. P. Semjonovs and P. Zikmanis. *Pediococcus pentosaceus* lactose-positive strain and a complex of fructan-containing exopolysaccharides synthesized by the strain. EP 2011859 A1.
16. Han Y. W. Microbial levan. Adv. Appl. Microbiol., 1990, 35:171-194.
17. Srikanth R., Reddy C. H.S.S.S., Siddartha G., Ramaiah M.J., Uppuluri K.B. Review on production, characterization and application of microbial levan. Carbohydrate Polymers, 2015, 120:102-114.
18. Patel A., Prajapati J.B. Food and Health Applications of Exopolysaccharides produced by Lactic acid Bacteria. Adv Dairy Res, 2013, 1:2.
19. Rehm B. H. A. Bacterial polymers: biosynthesis, modifications and applications. Nature Reviews Microbiology, (2010); doi:10.1038/nrmicro2354.
20. Szwengiel A., Czarnecka M., Roszyk H., Czarnecki Z. Levan production by Bacillus subtilis DSM 347 strain. EJPAU 2004, 7(2), [http:// http://www.ejpau.media.pl/].
21. Miller G. L. Use of Dinitrosalicylic Acid Reagent for Determination of Reducing Speciesr. Anal. Chem., 1959, 31 (3), pp 426-428. DOI: 10.1021/ac60147a030.
22. Muñoz-Gutiérrez I, Rodríguez-Alegría ME, López-Mungía A. Kinetic behavior and specificity of β-fructosidases in the hydrolysis of plant and microbial fructans. Process Biochem 2009, 44:891-898.
23. Ramón Pamies, José Ginés Hernández Cifre, María del Carmen López Martínez, José García de la Torre Determination of intrinsic viscosities of macromolecules and nanoparticles. Comparison of single-point and dilution procedures Colloid and Polymer Science, 2008, Volume 286, Number 11, p. 1223.
24. Bodis L. Quantification of spectral similarity: towards automatic spectra verification. PhD Thesis, ETH Zurich, 2007.
25. Nikonenko N.A. et al., Spectroscopic manifestation of stretching vibrations of glycosidic linkage in polysaccharides. J.Mol.Struct., 2005,v.752, pp.20-24.
26. EP 2 829 612 A1 (TECH UNIVERSITÄT MÜNCHEN [DE]) 28 January 2015 (2015-01-28).
27. HERMANN M ET AL: "Cultivation of selected Gluconobacter-strains on cereal substrates and in-situ production of exopolysaccharides", V SYMPOSIUM ON SOURDOUGH / CEREAL FERMENTATION FOR FUTURE FOODS; 10-12 OCTOBER 2012; HELSINKI, FINLAND (2012-10-10), page 87.
28. EP 2 876 169 A1 (UNIV LATVIJAS [LV]) 27 May 2015 (2015-05-27).

## Claims

1. An acetic acid bacterial strain *Gluconobacter nephelii,* which is deposited in the Microbial Strain Collection of Latvia under the number P 1464.

2. Products containing *Gluconobacter nephelii* strain P 1464 as per claim 1 and its produced extracellular fructan-type polysaccharides.

3. Use of *Gluconobacter nephelii* strain P 1464 for preparation of products according to claim 2.

## Patentansprüche

1. Essigsäure-Bakterienstamm *Gluconobacter nephelii*, der in der Mikrobenstamm-Sammlung von Lettland unter der Nummer P 1464 gelagert wird.

2. Produkte, die den *Gluconobacter nephelii*-Stamm P 1464 gemäß Anspruch 1 und seine produzierten, extrazellulären fructanähnlichen Polysaccharide enthalten.

3. Verwendung des *Gluconobacter nephelii*-Stamms P 1464 für die Herstellung von Produkten nach Anspruch 2.

## Revendications

1. Souche bactérienne d'acide acétique de *Gluconobacter nephelii*, qui est déposée à la Collection de souches microbiennes de Lettonie sous le numéro P 1464.

2. Produits contenant la souche de *Gluconobacter nephelii* P 1464 selon la revendication 1 et ses polysaccharides extracellulaires de type fructane produits.

3. Utilisation de la souche de *Gluconobacter nephelii* P 1464 pour la préparation de produits selon la revendication 2.
